# EUROPÄISCHE PATENTSCHRIFT

(11) **EP 1 878 433 B1**
(45) Veröffentlichungstag und Bekanntmachung des Hinweises auf die Patenterteilung: **05.01.2011**
(21) Anmeldenummer: 06014711.3
(22) Anmeldetag: 14.07.2006
(51) Int. Cl.: A61K 33/00, A61K 35/00, A61P 17/00

(54) **Magmatithaltige topische Zusammensetzung**
Topical composition comprising magmatites
Composition topique contenant du magmatite

(43) Veröffentlichungstag der Anmeldung: 16.01.2008
(73) Patentinhaber: Colombini, Paolo, 38040 Fornace TN (IT)
(72) Erfinder: Colombini, Paolo, 38040 Fornace TN (IT)
(74) Vertreter: Vossius & Partner

(56) Entgegenhaltungen:
- EP-A2- 1 046 397
- WO-A-2004/024169
- US-A- 4 083 965
- DATABASE WPI Week 200162 Derwent Publications Ltd., London, GB; AN 2001-552471 XP002412199 & JP 2001 151682 A (KEIKOSHA YG) 5. Juni 2001 (2001-06-05)
- DATABASE WPI Week 198549 Derwent Publications Ltd., London, GB; AN 1985-307655 XP002412200 & JP 60 214720 A (OGIWARA H) 28. Oktober 1985 (1985-10-28)
- DATABASE WPI Week 199838 Derwent Publications Ltd., London, GB; AN 1998-440239 XP002412201 & JP 10 139671 A (KENYU KK) 26. Mai 1998 (1998-05-26)
- DATABASE WPI Week 199534 Derwent Publications Ltd., London, GB; AN 1995-261206 XP002412202 & JP 07 165591 A (EARTH CLEAN KK) 27. Juni 1995 (1995-06-27)
- DATABASE WPI Week 200565 Derwent Publications Ltd., London, GB; AN 2005-632593 XP002412203 & JP 2005 179331 A (FUKIDANI T) 7. Juli 2005 (2005-07-07)
- DATABASE WPI Week 200441 Derwent Publications Ltd., London, GB; AN 2004-435517 XP002412204 & JP 2004 161723 A (MAMUZU JAPAN KK) 10. Juni 2004 (2004-06-10)
- DATABASE WPI Week 200262 Derwent Publications Ltd., London, GB; AN 2002-581419 XP002412205 & RU 2 182 010 C2 (BRYUZINA N P) 10. Mai 2002 (2002-05-10)
- DATABASE WPI Week 200438 Derwent Publications Ltd., London, GB; AN 2004-401595 XP002412206 & CN 1 483 419 A (CHANG X) 24. März 2004 (2004-03-24)
- DATABASE WPI Week 200634 Derwent Publications Ltd., London, GB; AN 2006-319239 XP002412207 & CN 1 709 497 A (GUIZHOU MIAOYING PHARM SCI & TECH DEV CO LTD) 21. Dezember 2005 (2005-12-21)
- DATABASE WPI Week 200205 Derwent Publications Ltd., London, GB; AN 2002-035055 XP002412208 & CN 1 312 110 A (WANG F) 12. September 2001 (2001-09-12)
- DATABASE WPI Week 199530 Derwent Publications Ltd., London, GB; AN 1995-224804 XP002412209 & CN 1 087 266 A (GAO Y) 1. Juni 1994 (1994-06-01)

## Beschreibung

Die vorliegende Erfindung betrifft topische Zusammensetzung umfassend Gesteinsmehl oder Gesteinsschlamm von magmatischen Gesteinen und deren Verwendung zur Behandlung von Hauterkrankungen wie Psoriasis, Ekzemen, Neurodermatitis und Seborrhoe.

Psoriasis, zu deutsch Schuppenflechte ist eine familiär gehäuft vorkommende Hautkrankheit an der zwischen ein bis drei Prozent der Bevölkerung Europas und Amerikas leiden. Typisch für die Psoriasis sind scharf begrenzte, rote teils juckende erhabene mit silbrigen Schuppen bedeckte Herde.

Infolge von gesteigerter Proliferation der Epidermis kommt es hierbei zu starker Schuppenbildung. Oft entstehen nur einzelne Herde an den Streckseiten der grossen Gelenke, die Schuppenflechte kann sich aber auch auf den gesamten Körper ausbreiten.

Die Auslöser können hierbei vielfältig sein wie z.B. das Klima, mechanische, thermische oder chemische Irritationen, postexanthematisch, Stoffwechselstörungen, durch Einsetzen der Menarche, Gravidität, Laktation oder der Menopause, und psychische Belastung. Die Hautzeichen selbst werden ausgelöst durch einen fehlgesteuerten Angriff des Immunsystems (Autoimmunreaktion) gegen Zellen der Oberhaut (Keratinozyten), die sich als Reaktion darauf besonders stark vermehren. In der Folge wird die Haut an den betroffenen Stellen besonders dick und schuppig.

Psoriasis verläuft in Schüben oder chronisch, evtl. mit wechselnder Lokalisation u. Ausdehnung, bevorzugt an Ellbogen, Knien, behaartem Kopf, Handtellern u. Fußsohlen, Körperfalten, Nägeln (Tüpfelnägel, "Ölfleck", subunguale Hyperkeratosen). Sie tritt evtl. auch an Schleimhäuten und Gelenken auf, z.B. im Mund als Leukoplakie oder Exfoliatio areata linguae, beim Typ v. ZUMBUSCH mit weißl.-gelbl.-rötl. Erhebungen, Mikropusteln oder Erosionen mit Steigerung bis zu ektodermalen (erythrodermalen), mesenchymalen (P. arthropathica = Arthritis psoriatica) u. exsudativen Maximalformen (Psoriasis pustulosa).

Es werden somit drei Hauptformen der Psoriasis unterschieden, die gewöhnliche Schuppenflechte (Psoriasis vulgaris), die mit Eiterblässchen einhergehende Psoriasis pustulosa und die Psoriasis-Arthritis der Gelenke.

Bislang sind eine Vielzahl von Therapieansätzen zur Behandlung der Psoriasis beschrieben worden. Diese sind jedoch unbefriedigend, da sie regelmäßig mit rezidiven verbunden sind. So hängt die Auswahl der Therapie von der individuellen Situation ab und bedarf oft einer Kombination verschiedener Medikamente und (physikalischen)Therapien. Es gibt eine Vielzahl von Präparaten und physikalischen Therapieformen, die zu einer Besserung der Beschwerden und auch zur vorübergehenden Beschwerdefreiheit führen können, eine Heilung ist jedoch mit den heute zur Verfügung stehenden Mitteln nicht möglich.

Das Behandlungsspektrum reicht hierbei von hautpflegenden Salben bis hin zu starkwirksamen, das Immunsystem hemmenden Medikamenten .

Für die lokale Therapie werden die Wirkstoffe äusserlich in Form von Cremes, Salben, Lotionen oder auch Shampoos und Badezusätzen verwendet.

Bei einem akuten Schub muss zunächst die dicke Hornschicht durch Salbenverbände mit Wirkstoffen wie Salicylsäure, Harnstoff oder Milchsäure beseitigt werden, damit die eingesetzten Wirkstoffe zum Wirkort vordringen können. Zur Behandlung von Krankheitsschüben werden dann u.a. Vitamin D- und Cortisonpräparate sowie Salben mit Teerpräparaten, Cignolin (Dithranol) oder Retinoiden eingesetzt.

Bei schweren Fällen, insbesondere bei der Psoriasis arthropatica werden Immunsuppressiva, Zytostatika wie Methotrexat, Ciclosporin A oder Retinoide oral eingesetzt. Diese Medikamente sind aber durch ihre Teratogenität und ihre z.T. schweren Nebenwirkungen für eine Therapie von Frauen im gebärfähigen Alter und/oder eine Dauertherapie nicht geeignet.

Zudem muss auch in weitgehend beschwerdefreien Perioden die psoriatrische Haut durch z.B. harnstoffhaltige Pflegepräparate sorgfältig gepflegt werden.

Badetherapien mit Salzen aus dem Toten Meer in Kombination mit Sonnenbestrahlung (Foto(sole)therapie) oder die Photochemotherapie (PUVA) bei welcher ein Lichtsensibilisator (Psoralen) in Kombination mit UVA-Licht verwendet wird sind weitere Therapieansätze bei der Behandlung von Psoriasis und anderen Hauterkrankungen wie der Neurodermatitis unterstützend angewandt, verschaffen aber nur eine kurzfristige Erleichterung.

Da die zur Zeit verwendeten medikamentösen und anderen Therapien allenfallls eine kurzzeitige Linderung bewirken und/oder z.T. mit schweren Nebenwirkungen verbunden sind, besteht somit ein dringender Bedarf für ein sicheres und effizientes Mittel zur Behandlung von Psoriasis und ähnlichen Hauterkrankungen, welche u.a. auch bei grossflächigen Erkrankungen eingesetzt werden kann.

Magmatische Gesteine (Magmatite) zu welchen u.a. die Plutonite (Tiefengesteine) und die Vulkanite (Ergussgesteine) gehören zählen neben den Sedimentiten (Sedimentgesteinen) wie Sandstein und Dolormit und Metamorphiten (Metamorphen Gesteinen) wie Marmor zu den wichtigsten bodenbildenden Gesteinen.

Vulkanite werden weiter unterteilt in die Ryolith-Familie, Trachyt-Familie, Andesit/Basalt-Familie und die Pikrit-Familie. Plutonite lassen sich weiter unterteilen in die Quarzolith-Familie, Granit-Familie, Syenit-Familie, Diorit/Gabbro-Familie und Peridotit-Familie.

Beispiele für Magmatite sind u.a. Granit, Diorit, Gabbro, Syenit, Porphyr (Rhyolith), Quarzporphyr, Porphyrit, Quarzporphyrit, Basalt, Basaltuff, Tuffit, Bims, Andesit Lavalit, Melaphyr und Diabas sowie effusivers Porphyrgestein aus der Gruppe der quarzhaltigen Porphyre und intrusiver Granitgesteine aus der Gruppe der Graniten. Die obengenannten Gesteinsarten sind u.a. in Streckeisen, A. L.: To each Plutonic rock ist proper name, Earth Sci.Rev. 12, 1-33, Amsterdam, 1976, Villavecchia-Eigenmann, Nuovo Dizionario di Merceologia applicata, 1983, p.660; Ardito Desio, Geologia applicata all'ingegneria, 3. Auflage, Mailand, S. 24 ff.; Atlante della pietra trentina, Trento, 2005, S.133 u. S. 151; Bargosi G.M., Avanzini M., Mair V., Morelli C., Neri C., Sapelza A., The Monte duco Volcanic Sequence (Bolzano - Trento Area), in the Southern Alps, in "Stratigraphy and Facies of the Permian Deposits between Eastern Lombardy and the Western Dolomites - International Congress: The Continental Premian, 15-25 September 1999, Brescia, Italien; näher beschrieben und bezüglich ihrer.Entstehung und Zusammensetzung definiert.

Es wurde nun in Versuchen über 15 Monate überraschender Weise gefunden, dass der topische Kontakt mit einer Aufschlämmung von magmatithaltigem Gesteinsmehl (Gesteinsschlamm) wie Porphyrit oder Granit zur einer überraschenden Verbessung des Hautzustandes bis zur vollkommenden Heilung bei Psoriasis führt.

Der Gegenstand der vorliegenden Erfindung sind somit kosmetische und medizinische topische Zusammensetzungen umfassend magmatithaltiges Gesteinsmehl oder Gesteinsschlamm und die Verwendung von magmatithaltigem Gesteinsmehl oder Gesteinsschlamm zur Herstellung von topischen Zusammensetzung zur Behandlung von Hauterkrankungen, wie Psoriasis, Ekzemen, Neurodermatitis und Seborrhoe.

Besonders bevorzugt ist hierbei die Behandlung von Psoriasis.

Magmantit wie im Zusammenhang mit der vorliegenden Erfindung verwendet bezeichnet sowohl Vulkanite als auch Plutonite, besonders bevorzugt sind hierbei Gesteine aus der Gruppe der Porphyre und Granite Ursprung.

Das Gesteinsmehl bzw. die Aufschlämmung des Gesteinsmehls fällt üblicherweise bei der Bearbeitung der obengenannten Gesteine in einem Steinmetzbetrieb an. Die Grösse der Pulverpartikel und bzw. der aufgeschlämmten Partikel (Schlämme) betragen durchschnittlich weniger als 40 Mikron, insbesondere bei Porphyr und Granit.

Magmatite bestehen u.a. aus Siliciumdioxid (SiO₂) und werden je nach SiO₂ - Gehalt grundlegend in drei Gruppen unterteilt. Bei einem SiO₂-Gehalt von weniger als 52 % spricht man basaltischen Magmatiten, hierzu gehören Basalt und Gabbro, bei einem SiO₂-Gehalt von 52 % bis 65 % spricht man von intermediären Magmatiten, hierzu gehören Andesit und Diorit, ein SiO₂-Gehalt von mehr als 65 % kennzeichnet die sauren Magamtite, wie Porphyr (Ryolith), Quarzporphyr und Granit.

Erfindungsgemäss bevorzugt wird die Verwendung von Gesteinsmehl oder Gesteinsschlamm von saurem Magmatiten, wie Granit , Quarzporphyr oder Porphyr (Ryolith).

Die chemische Zusammensetzung von besonders Porphyr- und Granitgesteinen, bzw der durch die Bearbeitung erhaltenen besonders bevorzugten Gesteinsmehle und -schlämme sind in Tabelle A beschrieben.

**Tabelle A**

| | | | |
|---|---|---|---|
| SiO₂ | > | 65,00 | % |
| Al₂O₃ | > | 12,00 | % |
| TiO₂ | > | 0,15 | % |
| FeO | > | 0,50 | % |
| Fe₂O₃ | > | 0,65 | % |
| MgO | > | 1,00 | % |
| CaO | > | 1,00 | % |
| K₂O | > | 4,00 | % |
| Na₂O | > | 3,50 | % |

Die erfindungsgemässe topische Zusammensetzung kann in jeder dem Fachmann bekannten Form die für die topische Applikation geeignet ist vorliegen, wie z.B. als Schüttelmixtur/Schüttelpinselung, wässrige, ölige oder alkoholische Suspension, Emulsion, Paste, Creme, Salbe, lipophiles Gel, hydrophiles Gel oder Pflaster, auch die Formulierung als medizinisches Shampoo, Badezusatz oder Schlamm ist möglich.

Die erfindungsgemässen Zusammensetzungen können in für den Fachmann bekannter Art und Weise hergestellt und formuliert werden, z.B. wie in Remington's Pharmaceutical Sciences, 15 Aufl. Mack Publishing Co., New Jersey (1991) und Bauer et al, Pharmazeutische Technologie, 5. Aufl., Govi-Verlag Frankfurt, 1997 beschrieben.

Bestandteile die bei der Formulierung der erfindungsgemässen Zusammensetzungen verwendet werden können umfassen z.B. (thixotrope) hydrophile Konisistenzgeber wie Methyl-, Hydroxypropylcellulose, Xanthangummi, Polyacrylsäuren, Stärke, Gelatine, Alginate, Kieselsäure, Magnesiumaluminiumsilikat, Bentonit; (thixotrope) lipophile Konsistenzgeber wie Wollwachs, Glycerolmonostearat, Cetylpalmitat, Ethylstearylalkohol, Bienenwachs und Hartparaffin; Verdünnungsmittel bzw. Dispersionsmittel wie Wasser, einwertige Alkohole wie Ethanol, Isopropanol oder deren Mischungen und mehrwertige Alkohole wie Glycole; fette Öle wie Sojaöl, Kokosnussöl, Erdnussöl, Avocadoöl, Nachtkerzenöl, Olivenöl, Baumwollsaatöl, Saffloröl; Ethyloleat, Isopropylmyristat und Oleyloleat; Siliconöle wie Dimethicon; und flüssige oder halbfeste Kohlenwasserstoffe wie Vaseline; Hartparaffin, Ceresin, Mineralöle bzw. Paraffinöle oder niedrigschmelzende Wachse, Solubilisierungsmittel; Stabilisierungsmittel; Puffer, Emulgatoren und Tenside, wie Fettsäureester wie z.B. Laureth-4, Steareth-2, Ceteareth-12, Oleth-5 oder Sorbitanfettsäureester wie z.B. Sorbitanmonolaurat, -stearat, oder Polysorbat 20, 40 oder 60; Polyglycerylester, Zuckerester oder Rizinusölverbindungen; Benetzungsmittel, Feuchthaltemittel wie Glycerin, Propylenglycol, Hexylenglycol, Butandiol und Sorbitol; Fettsäuren/Fettalkohole; Farbstoffe, Konservierungsmittel wie Benzoe-, Sorbinsäure, Pheoxyethanol, Benzylalkohol, Parabene und Benzalkoniumchlorid; und Antioxidantien wie alphatocopherol. Ascorbinsäure, Butylhydroxytoloul, Propyl-, Dodecylgallat sowie Synergisten wie Citronensäure oder EDTA; Feststoffe wie Talkum, Titan(IV)oxid, Mais-, Reisstärke oder hochdisperses Siliciumdioxid; Neutralisationsmittel wie Natronlauge, Triethanolamin oder Trometamol; Filmbildner wie Polyvidon oder PVP-Copolymere und Penetrationsförderer wie Diethylenglycolmonoethylether.

Der Fachmann kann z.B. somit bekannte halbfeste lipophile und hydrophile Grundlagen zur Formulierung der erfindungsgemässen Zusammensetzungen wie z.B. Kohlenwasserstoffgrundlagen (wie weißes oder gelbes Vaselin Ph. Eur., Kunstvaseline, Naturvaseline Triglyceride, Neutralöle, gelbes Wachs, gebleichtes Wachs, Polyalkylsiloxane, dick- und dünnflüssige Parafine, Hartparafin, Plastibase), Macrogol bzw. Polyethylenglycolsalben, hydrophobes Basisgel DAC, Schweineschmalz DAB, Halbfestes Hartfett Ph. Eur. (z.B. Softisan 378, (wasserhaltige) Wollwachsalkoholsalbe, (wasserhaltige) Hydrophile Salbe des DAB, Basiscreme DAC, Oleogele und Hydrogele verwenden.

Zudem können die erfindungsgemässen Zusammensetzungen feuchtigkeitsbindende Stoffe wie Harnstoff enthalten.

Auch hydrophile Grundlagen z.B. mit Carboxymethylcellulose als Gelbildner können erfindungsgemäss verwendet werden.

Für Suspensionen und Schüttelmixturen/Schüttelpinselungen sind Wasser und Alkohol/Wassermischungen aus Wasser und Ethanol oder Isopropanol als Dispersionsmittel geeignet. Als Grundlagen für ölige Suspensionen und Schüttelmixturen werden insbesondere fette Öle wie Olivenöl, Erdnussöl, Nachtkerzenöl oder Kokosnussöl verwendet.

Als Grundlage für Pasten eignet sich insbesondere Pflanzen(Birken)teer.

Der Gehalt der topischen Zusammensetzungen an Gesteinsmehl beträgt von vorzugsweise 10 % bis 50 %, insbesondere bevorzugt ist ein Gehalt von 40 %.

Weitere pharmazeutisch verträgliche Exzipienten/Bestandteile sind u.a. in Remington's Pharmaceutical Sciences, 15. Aufl., Mack Publishing Co., New Jersey (1991) und Bauer et al, Pharmazeutische Technologie, 5. Aufl., Govi-Verlag Frankfurt, 1997 beschrieben.

In einer weiteren bevorzugten Ausführungsform enthalten die erfindungsgemässen Zusammensetzungen zusätzlich Birkenteer (Pix betulae, Pflanzenteer) insbesondere in einer Menge 10 % bis 70 %, besonders bevorzugt in einer Menge von 60 %.

Alle Prozentangaben beziehen sich auf % Gew./Gew.

### Formulierungsbeispiel

| Paste | |
|---|---|
| Gesteinsmehl | 40 g |
| Pflanzenteer | 60 g |

Das Gesteinsmehl wird allmählich mit dem gegebenenfalls erwärmten Pflanzenteer zu einer gleichmässigen Masse angerieben und falls erforderlich mit einer Salbenmühle homogenisiert.

## Patentansprüche

1. Zusammensetzung zur topischen Anwendung umfassend magmatithaltiges Gesteinsmehl und 10 - 70 % Pflanzenteer.

2. Zusammensetzung nach Anspruch 1, wobei das Gesteinsmehl ausgewählt ist aus Granit, Diorit, Gabbro, Syenit, Porphyr (Rhyolith), Porphyrit, Basalt, Basalttuff, Tuffit, Bims, Lavalit, Melaphyr und Diabas, sowie effusivem Porphyrgestein aus der Gruppe der quarzhaltigen Porphyre und intrusiver Granitgesteine aus der Gruppe der Graniten.

3. Zusammensetzung nach Anspruch 1 oder 2 in Form einer Schüttelmixtur/Schüttelpinselung, Suspension, Emulsion, Paste, Creme, Salbe, eines Gels, Pflasters, medizinischen Shampoos, Badezusatzes oder Schlamms.

4. Zusammensetzung nach einem der Ansprüche 1 bis 3 zur Therapie von Psoriasis, Ekzemen, Neurodermatitis oder Seborrhoe.

5. Zusammensetzung nach einem der Ansprüche 1 bis 4, zusätzlich enthaltend Harnstoff.

6. Zusammensetzung nach einem der Ansprüche 1 bis 5, zusätzlich umfassend mindestens einen weiteren Wirkstoff ausgewählt aus Psoralen, Corticosteroiden, Retinoiden, Toten-Meer-Salzen, Cignolin, Salicylsäure und Milchsäure.

## Claims

1. A composition for topical use comprising powdered mineral containing magmatite and 10 - 70% vegetable tar.

2. The composition of claim 1 wherein the powdered mineral is selected from granite, diorite, gabbro, syenite, porphyry (rhyolite), porphyrite, basalt, basaltic tuff, tuffite, pumice, lavalit, melaphyre and diabase as well as effusive porphyry rock of the group of quartzous porphyries and intrusive granite rocks of the group of granites.

3. The composition of claim 1 or 2 in form of a mixture to be shaken/brushing mixture to be shaken, suspension, emulsion, paste, cream, ointment, a gel, plaster, medical shampoo, bath salt or mud.

4. The composition of any one of claims 1 to 3 for the therapy of psoriasis, eczemas, neurodermatitis or seborrhea.

5. The composition of any one of claims 1 to 4, further containing urea.

6. The composition of any one of claims 1 to 5, further comprising at least a further active ingredient selected from psoralen, corticosteroids, retinoids, Dead Sea salts, cignolin, salicylic acid and lactic acid.

## Revendications

1. Composition pour application topique, comprenant de la poudre de roche contenant de la magmatite et 10 à 70 % de goudron végétal.

2. Composition selon la revendication 1, dans laquelle on choisit la poudre de roche dans le groupe constitué du granite, de la diorite, du gabbro, de la syénite, du porphyre (rhyolite), de la porphyrite, du basalte, du tuf basaltique, de la tuffite, de la pierre ponce, de la lavalite, de la mélaphyre et de la diabase, ainsi que de la roche porphyrique effusive du groupe des porphyres contenant du quartz et des roches granitiques intrusives du groupe des granites.

3. Composition selon la revendication 1 ou 2 sous la forme d'une lotion/d'un badigeon, d'une suspension, d'une émulsion, d'une pâte, d'une crème, d'une pommade, d'un gel, d'un timbre transdermique, d'un shampooing médical, d'un additif de bain ou d'une boue.

4. Composition selon l'une quelconque des revendications 1 à 3 pour traiter le psoriasis, les eczémas, la neurodermatite ou la séborrhée.

5. Composition selon l'une quelconque des revendications 1 à 4, contenant en outre de l'urée.

6. Composition selon l'une quelconque des revendications 1 à 5, comprenant en outre une autre substance active choisie parmi le psoralène, les corticostéroïdes, les rétinoïdes, les sels de la mer Morte, la cignoline, l'acide salicylique et l'acide lactique.
